# EUROPEAN PATENT SPECIFICATION

(11) **EP 0 854 143 B1**
(45) Date of publication and mention of the grant of the patent: **07.11.2001**
(21) Application number: 98100806.3
(22) Date of filing: 19.01.1998
(51) Int. Cl.: C07D 309/18, C07D 309/20

(54) **Diels-Alder reaction of aldehydes with simple dienes**
Diels-Alder Reaktion von Aldehyden mit einfachen Dienen
Réaction de Diels-Alder d'aldéhydes avec des diènes simples

(30) Priority: 21.01.1997 EP 97300377
(43) Date of publication of application: 22.07.1998
(73) Proprietor: QUEST INTERNATIONAL B.V., 1411 GP Naarden (NL)
(72) Inventor: Newman, Christopher Paul, Canterbury, Kent CT1 3NT (GB); Aggarwal, Varinder Kumar, Sheffield S10 2ST (GB); Vennall, Graham Patrick, East Sussex TN39 4DA (GB)
(74) Representative: Matthews, Heather Clare

(56) References cited:
- EP-A- 0 325 000
- US-A- 4 709 085
- US-A- 4 847 392
- PATENT ABSTRACTS OF JAPAN vol. 097, no. 011, 28 November 1997 & JP 09 176171 A (NOGUCHI KENKYUSHO; ASAHI CHEM. IND. CO., LTD.)
- J. INANAGA ET AL.: NEW J. CHEM., vol. 19, 1995, pages 707-712, XP002049944
- H. GRIENGL ET AL.: MONATSHEFTE FUR CHEMIE., vol. 107, 1976, WIEN AT, pages 675-684, XP002063877
- M.F. ANSELL ET AL.: JOURNAL OF THE CHEMICAL SOCIETY, CHEMICAL COMMUNICATIONS., 1972, LETCHWORTH GB, pages 739-740, XP002063878
- S. OI ET AL.: TETRAHEDRON LETTERS, vol. 37, no. 35, 1996, OXFORD GB, pages 6351-6354, XP002063879
- CHEMICAL ABSTRACTS, vol. 91, no. 7, 13 August 1979 Columbus, Ohio, US; abstract no. 56685y, XP002063880 & G.A. OLAH ET AL.: SYNTHESIS, vol. 4, 1979, STUTTGART DE, pages 270-271,
- CHEMICAL ABSTRACTS, vol. 126, no. 26, 30 June 1997 Columbus, Ohio, US; abstract no. 343460t, XP002063881 & V.K. AGGARWAL ET AL.: TETRAHEDRON LETTERS, vol. 38, no. 14, 1997, OXFORD GB, pages 2569-2572,
- CHEMICAL ABSTRACTS, vol. 127, no. 9, 1 September 1997 Columbus, Ohio, US; abstract no. 121610y, XP002063882 & T. HANAMOTO ET AL.: BULLETIN OF THE CHEMICAL SOCIETY OF JAPAN., vol. 70, no. 6, 1997, TOKYO JP, pages 1421-1426,

## Description

The invention relates to catalysed Diels-Alder reactions between non-activated aldehydes and simple dienes leading to dihydropyrans.

The hetero Diels-Alder (D-A) reaction between aldehydes and dienes has been limited mainly to the reaction of either highly electron rich dienes (Danishefsky's and similar dienes) with a broad range of aldehydes, or of highly activated aldehydes (e.g. chloral and glyoxylates) with a broad range of dienes, see e.g. H Waldmann, Synthesis 1994, 535. Recently the reaction between simple dienes and unactivated aldehydes has been achieved using a cationic Pd²⁺ catalyst, see: S. Oi et al, Tet. Letters., 37 (1996), 6351. Furthermore, the Lewis acid catalysed D-A reaction of non-activated aldehydes and simple dienes has been described in US 5,162,551 in which a mixture of AlCl₃ or SnCl₄ with nitroalkanes (particularly 2-nitropropane) is used. The reported yields are good, but the catalyst system is potentially explosive and difficult to handle. J. Inanaga. New J. Chem. 1995 19, 707 reported the use of lanthanide (III) salts of superacids such as trifluoromethanesulfonic acid (triflic acid, TfOH) and perfluorooctanesulfonic acid (PfOH) as catalysts in D-A reactions. He reports that with 30 mole% of Yb(OPf)₃ and Sc(QPf)₃ high yields of 2-phenyl-4,5-dimethyl-2,3-dihydropyran were obtained from benzaldehyde and 2,3-dimethyl-butadiene. With Sc(OTf)₃ only moderate yields were obtained.

Furthermore, various reactions between simple aliphatic dienes and non-activated aldehydes catalysed by strong Brönsted acids and leading to 5,6-dihydropyrans have been reported as outlined below. This reaction has also been categorized as a Prins-type reaction.
i AU 13822/70: formaldehyde + 1,3-butadiene with sulphuric acid;
ii M.F. Ansell and A.A. Charalambides, J.C.S. Chem. Comm., 1972, 739: benzaldehyde + 2,3-dimethyl-butadiene with p-toluene-sulphonic acid at 80°C;
iii H. Griengl and K.P. Geppert, Monatsh. Chem. 107 (1976), 675: 1,3-butadiene, isoprene or 2,3-dimethyl-1,3-butadiene + benzaldehyde with concentrated sulphuric acid.

In i very low yields were reported, in ii the reaction had to be carried out at 80°C and in iii a tenfold excess of aldehyde was used and moreover, the reaction with isoprene and with 1,3-butadiene produced a complicated reaction mixture.

The D-A reaction between non-activated aromatic aldehydes and simple aliphatic dienes is particularly useful for preparing dihydropyrans. Examples are the fragrance materials 6-phenyl-4-methyl-5,6-dihydropyran, also known as Rosyrane, 6-phenyl-2,4-dimethyl-dihydropyran and 6-butyl-2,4-dihydropyran. Rosyrane is itself a starting material for the preparation of the fragrance material Mefrosol, also known as Phenoxanol, through reductive ringopening in acidic media (see US 5,162,551).

Although the use of the above mentioned lanthanide salts would appear to give good prospects for such D-A reactions, lanthanide salts are expensive, a high molar ratio of catalyst (30 mole%) appears to be required and lanthanide salts of perfluorooctanesulfonic acid are high molecular weight materials. The latter means that high amounts of solid material need to be handled which adds considerably to the production costs.

It has now been found that non-activated aldehydes RCHO, in which R is a C1-C10 aliphatic group or phenyl group which is unsubstituted or substituted with one or more C1-C5 alkyl groups or one other functional group, may be reacted in a D-A reaction with simple aliphatic dienes R₁CH=CR₂-CR₃=CHR₄, in which R₁ - R₄ are H or C1-C5 alkyl groups, using as a catalyst a perfluorinated organic sulphonic acid or a Brönsted acid derivative thereof.

Thus, the invention provides a process for the preparation of 5,6-dihydropyrans of the general formula. 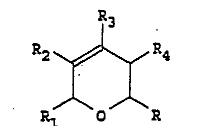 in which the groups R and R₁-R₄ have the meaning given above, comprising reacting the corresponding aldehyde with the corresponding diene under the influence of a perfluorinated organic sulphonic acid or a Bronsted acid derivative thereof. Preferred are perfluorooctanesulphonic acid, triflic acid, polymeric perfluoroalkanesulfonic acids such as those known as Nafion*, as well as Bronsted acid derivatives of triflic acid, particularly trifluoromethanesulfonimide (triflimide). The acids can be pure, but need not necessarily be water-free.
* Trademark of Dupont de Nemours of Wilmington Delaware

Preferably the groups R₁-R₄ are H or methyl, more preferably at most two of R₁-R₄ are methyl and the others are hydrogen. Particularly preferred dienes are isoprene, 2,3-dimethylbutadiene, 1,3-dimethyl-butadiene and 1,3-pentadiene.

R is preferably a C2-C6 aliphatic group or a phenyl group which is unsubstituted or mono-substituted with C1-C3 alkyl or with 4-Cl, 4-nitro or 3-methoxy. Particularly preferred phenyl groups are: phenyl and 4-methylphenyl.

The aldehyde/diene ratio does not have much influence on the course of the reaction. Ratios between 10:1 and 1:20 are suitable and between 5:1 and 1:10 are preferred. In practice an about equimolar ratio or slight excess of diene works very well.
The amount of catalyst is preferably chosen between 0.01 and 50 mol% based on the reactant being present in lowest molar amount, more preferably between 0.1 and 20%, most preferably between 0.2 and 15%.
The reaction is conveniently carried out in an organic solvent. This does not necessarily have to be completely water-free, as would be necessary with many Lewis acid catalysts. Preferred are low polarity or non-polar solvents; particularly preferred are hydrocarbons such as hexane, cyclohexane, toluene and petroleum spirit.

The reaction is conveniently carried out above -10°C and below +80°C, more preferably between 0 and 60°C, most preferably between 10 and 40°C. Ambient reaction temperatures and pressures are most convenient from a practical point of view, although higher pressures may sometimes be useful, e.g. with volatile dienes. Since the reaction is not very exothermic no expensive measures have to be taken to keep the reaction temperature within the desired limits.

The reaction proceeds regioselectively, leading predominantly to one positional isomer if an unsymmetric diene is used. Thus, the reaction between benzaldehyde and isoprene leads to 4-methyl-6-phenyl-5,6-dihydropyran. Some double bond isomerisation may take place leading to the presence of the 4-methylene- and/or the 2,3-dihydro-isomers in the reaction product

The reaction mixtures can be worked up in the usual way and the reaction product isolated and purified by conventional means such as distillation, chromatographic techniques and the like. Non-aqueous work-up is particularly convenient as it reduces the amount of waste water.

### Example 1

### Preparation of 4-methyl-6-phenyl-5,6-dihydropyran.

To a rapidly stirred solution of 70% aqueous triflic acid (2.10g, 0.01 mol) and benzaldehyde (106.1g, 1.0 mol) in 100ml toluene was added isoprene (150 cm³, 1.5 mol) over 4 hrs. at 25°C. The mixture was stirred at room temperature for a further 11 hours after which the reaction mixture was quenched by the addition of Polyrad* (2g) and solid sodium carbonate (5g). The crude mixture was distilled under reduced pressure to give pure rosyrane (56% yield) as a colorless oil.
* Trademark of Hercules for polyethoxylated abietyl amine.

### Examples 2-6

Under the same conditions the following aldehydes and dienes were reacted, leading to the indicated yields of the corresponding dihydropyran (GC detected yield given):

| Aldehyde | Diene | Yield (%) |
|---|---|---|
| benzaldehyde | isoprene | 72 |
| 4-methyl-benzaldehyde | " | 65 |
| 4-Cl-benzaldehyde | " | 68 |
| benzaldehyde | 2,3-dimethylbutadiene | 85 |
| " | 1,3-dimethylbutadiene | not det. |

### Example 7

Bis-trifluoromethanesulphonimide (70,5mg, 0.25mmol) was stirred in hexane (4ml) at room temperature. Benzaldehyde (510*µ*l, 5.0mmol) and isoprene (750*µ*l, 7.5mmol) were added and the mixture stirred at room temperature overnight. Water (5ml) was added and the aqueous layer was extracted with hexane (3x3ml). The combined organic layers were filtered through celite and analysed by glc. A yield of 42% of 4-methyl-6-phenyl-5,6-dihydropyran was observed.

## Claims

1. Process for the preparation of 5,6-dihydropyrans of the general formula: 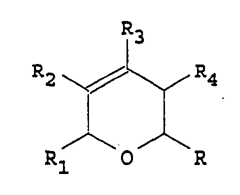 in which R is a C1-C10 aliphatic group or phenyl group which is unsubstituted or substituted with one or more C1-C5 alkyl groups or one other functional group and in which R₁-R₄ are H or C1-C5 alkyl groups, comprising reacting the corresponding aldehyde RCHO with the corresponding diene R₁CH=CR₂-CR₃=CHR₄ under the influence of a perfluorinated organic sulphonic acid or a Brönsted acid derivative thereof.

2. Process according to claim 1 wherein the perfluorinated organic sulfonic acid is perfluoro-octanesulphonic acid, triflic acid, Nafion or a Brönsted acid derivative of triflic acid.

3. Process according to claim 2 wherein the Brönsted acid derivative of triflic acid is bis-trifluoromethane-sulfonimide.

4. Process according to any one of claims 1-3. wherein R is a C2-C6 aliphatic group or a phenyl group which is unsubstituted or mono-substituted with C1-C3 alkyl or with 4-Cl, 4-nitro or 3-methoxy.

5. Process according to any one of claims 1-4 wherein the groups R₁-R₄ are H or methyl, preferably at most two of R₁-R₄ are methyl and the others are hydrogen.

6. Process according to any one of claims 1-5 wherein the aldehyde/diene ratio is between 10:1 and 1:20, preferably between 5:1 and 1:10.

7. Process according to any one of claims 1-6 wherein the amount of catalyst is between 0.01 and 50 mol% based on the reactant being present in the lowest molar amount, preferably between 0.1 and 20%.

## Patentansprüche

1. Verfahren zur Herstellung von 5,6-Dihydropyranen der allgemeinen Formel: 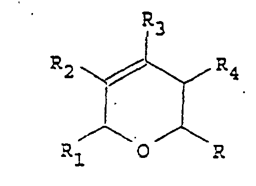 in der R eine C₁-C₁₀-aliphatische Gruppe oder Phenylgruppe ist, die unsubstituiert oder substituiert mit einer oder mehreren C₁-C₅-Alkylgruppen oder einer anderen funktionellen Gruppe ist, und in der R₁-R₄ H oder C₁-C₅-Alkylgruppen sind, umfassend das zur Reaktion bringen des entsprechenden Aldehyds RCHO mit dem entsprechenden Dien R₁CH=CR₂-CR₃=CHR₄ unter dem Einfluß einer perfluorierten organischen Sulfonsäure oder eines Brönsted-Säure-Derivats davon.

2. Verfahren gemäß Anspruch 1, wobei die perfluorierte organische Sulfonsäure Perfluoroctansulfonsäure, Triflinsäure, Nafion oder ein Brönsted-Säure-Derivat der Triflinsäure ist.

3. Verfahren gemäß Anspruch 2, wobei das Brönsted-Säure-Derivat der Triflinsäure Bis-trifluormethansulfonimid ist.

4. Verfahren gemäß mindestens einem der Ansprüche 1 bis 3, wobei R eine C₂-C₆-aliphatische Gruppe oder eine Phenylgruppe ist, die unsubstituiert oder monosubstituiert ist mit C₁-C₃-Alkyl oder mit 4-Cl, 4-Nitro oder 3-Methoxy.

5. Verfahren gemäß mindestens einem der Ansprüche 1 bis 4, wobei die Gruppen R₁-R₄ H oder Methyl sind, vorzugsweise höchstens zwei der R₁-R₄ Methyl und die anderen Wasserstoff sind.

6. Verfahren gemäß mindestens einem der Ansprüche 1 bis 5, wobei das Aldehyd/Dien-Verhältnis zwischen 10:1 und 1:20, vorzugsweise zwischen 5:1 und 1:10, liegt.

7. Verfahren gemäß mindestens einem der Ansprüche 1 bis 6, wobei die Menge des Katalysators zwischen 0,01 und 50 Mol%, bezogen auf den in der geringsten molaren Menge vorliegenden Reaktanten, beträgt, vorzugsweise zwischen 0,1 und 20%.

## Revendications

1. Procédé pour la préparation de 5,6-dihydropyrannes de formule générale : 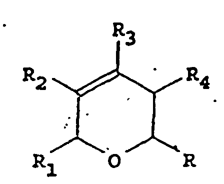 dans laquelle R représente un groupe aliphatique en C₁-C₁₀ ou un groupe phényle qui est non substitué ou substitué par un ou plusieurs groupes alkyle en C₁-C₅ ou un autre groupe fonctionnel, et dans laquelle R₁-R₄ représentent H ou des groupes alkyle en C₁-C₅, qui comprend la réaction de l'aldéhyde correspondant RCHO avec le diène correspondant R₁CH=CR₂-CR₃=CHR₄ sous l'influence d'un acide sulfonique organique perfluoré ou un de ses dérivés acide de Brönsted.

2. Procédé selon la revendication 1, dans lequel l'acide sulfonique organique perfluoré est l'acide perfluorooctanesulfonique, l'acide triflique, Nafion ou un dérivé acide de Brönsted de l'acide triflique.

3. Procédé selon la revendication 2, dans lequel le dérivé acide de Brönsted de l'acide triflique est le bis-trifluorométhanesulfonimide.

4. Procédé selon l'une quelconque des revendications 1-3, dans lequel R représente un groupe aliphatique en C₂-C₆ ou un groupe phényle qui est non substitué ou monosubstitué par un groupe alkyle en C₁-C₃ ou par 4-Cl, 4-nitro ou 3-méthoxy.

5. Procédé selon l'une quelconque des revendications 1-4, dans lequel les groupes R₁-R₄ représentent H ou un groupe méthyle, et dans lequel de préférence au plus deux d'entre R₁- R₄ représentent des groupes méthyle et les autres représentent des atomes d'hydrogène.

6. Procédé selon l'une quelconque des revendications 1-5, dans lequel le rapport aldéhyde/diène est compris entre 10:1 et 1:20, de préférence entre 5:1 et 1:10.

7. Procédé selon l'une quelconque des revendications 1-6, dans lequel la quantité de catalyseur est comprise entre 0,01 et 50 % en moles, par rapport au corps réagissant présent dans la plus faible quantité molaire, de préférence entre 0,1 et 20 %.
